# EUROPEAN PATENT SPECIFICATION

(11) **EP 2 598 962 B1**
(45) Date of publication and mention of the grant of the patent: **01.06.2016**
(21) Application number: 11732383.2
(22) Date of filing: 13.07.2011
(51) Int. Cl.: G05B 19/10

(54) **CONTROL DEVICE AND METHOD OF OPERATING SUCH A CONTROL DEVICE**
STEUERVORRICHTUNG UND VERFAHREN FÜR DEN BETRIEB EINER DERARTIGEN STEUERVORRICHTUNG
DISPOSITIF DE COMMANDE ET PROCÉDÉ DE MISE EN OEUVRE D'UN TEL DISPOSITIF DE COMMANDE

(30) Priority: 29.07.2010 EP 10007954
(43) Date of publication of application: 05.06.2013
(73) Proprietor: orangedental GmbH & Co. KG, 88400 Biberach (DE)
(72) Inventor: LAXHUBER, Ludwig, 82211 Herrsching (DE); PIERNITZKI, Bernhard, 19089 Demen (DE)
(74) Representative: Schollweck, Susanne
(86) International application number: PCT/EP2011/003502
(87) International publication number: WO 2012/013299

(56) References cited:
- EP-A1- 1 462 906
- WO-A1-2010/054150
- WO-A2-2007/084605
- US-A1- 2010 001 948

## Description

### TECHNICAL FIELD OF THE INVENTION

The present invention relates to a control device and in particular to a foot control device for the operation and control of medical or dental equipment or instruments. Moreover, the present invention relates to a method of operating such a control device.

### BACKGROUND OF THE INVENTION

Dental or medical professionals and practitioners use many instruments that are controlled by foot control systems. For example, surgical cutting instruments, endoscopic tools, irrigation and aspiration tools, dental drills and other handpieces, ultrasonic dental sealers, and dental prophylaxis units can be activated by means of foot control systems. A foot control system typically includes a foot control device or switch that is placed on the floor within easy reach of the practitioner. The foot switch is used to activate a dental/medical apparatus, which can include an operating base unit in communication with the foot switch. The foot switch Is typically connected to the base unit by a connector cable in a "hardwired" system. Alternatively, remote, "wireless" foot control systems, which do not use a connector cable, can be used to activate the base unit in some instances. A flexible, instrument cable connects the dental/medical instrument, for example, a dental handpiece, to the base unit. The dental or medical practitioner activates the base unit and the dental/medical instrument connected thereto by depressing the foot switch with his or her foot.

Some conventional foot switches are referred to as multi-position or multi-operation switches, i.e. switches that can control or trigger more than one function of an instrument in communication therewith. An operator depresses the pedal of the foot switch to a certain position, and this action causes the dental/medical instrument to operate in a specific mode. The particular operational mode is based on the position of the foot switch pedal. For example, with a two-position foot switch, a dental practitioner can depress the pedal to a first position so that water flows through the handpiece for rinsing the teeth of a patient. Then, the pedal of the foot switch can be depressed to a second position so that a cleaning spray flows through the handpiece for cleaning the teeth.

Foot control systems provide several advantages. First, the foot switch device is easy to use and efficient. The dental/medical professional or practitioner can activate the instrument in communication with the foot switch and optionally a base unit by simply depressing the foot switch with his or her foot. Secondly, the dental/medical practitioner's hands are kept free when working with a foot switch device. The practitioner thus can handle other instruments and accessories while treating the patient. Thus, the practitioner Is better able to concentrate on performing the required dental/medical procedure.

Foot switch devices can have a wide variety of structures. For example WO 07/084605 discloses a foot switch device for activating a dental or medical treatment Instrument. The foot switch device includes a base plate, a central housing attached to the base plate, an upper, moveable cover mounted on the housing, and a connecting collar attached to the upper cover for retaining the cover on the housing while allowing the cover to move upwardly and downwardly relative to the housing. The central housing contains a first electrical switch for transmitting a first signal to the instrument, and a second electrical switch for transmitting a second signal to the instrument. An operator depresses the upper cover with his or her foot to activate the switching mechanism and control the operation of the dental or medical instrument.

EP 1462906 discloses a foot switch device or regulator, especially for dental equipment. The foot regulator comprises a base part relative to which a regulating part of the foot regulator may be rotated and/or displaced in a radial direction, as well as means for detecting rotation and/or displacement. Moreover, the foot regulator comprises means for detecting the instantaneous position of the regulator, means for detecting relative movement relative to this position as well as detectable reference means for determining position and/or movement, wherein the detectable reference means for determining position and/or movement are formed by a pattern, diagram or elevations having a form of lattice structure. EP 1462906, moreover, discloses a method of controlling dental equipment, wherein the control is performed on the basis of a reading or detection of the instantaneous position of the foot regulator relative to a given zero point. The control takes place by rotation and/or radial displacement of one or more regulating rings.

The above described as well as other conventional foot switch devices that provide for more than one operation mode, i.e. can activate more than one function of an instrument in communication therewith, have the following drawback. Often, a dental/medical professional or practitioner, who is either standing on the ground or sitting on a chair, will have to change his location with respect to a patient, for instance, in order to obtain a different view of a region being examined or to examine different body parts of the patient. In doing so the dental/medical professional in a lot of cases will also change his position with respect to a foot switch located at a certain position on the floor. In order to still be able to operate any dental/medical instrument or equipment controlled by the foot switch the dental/medical professional will have to adjust the position of the foot switch to be accessible from his current position. Often the dental/medical professional achieves this by "dragging" the foot switch device along the floor by using his foot. In doing so the relative angular position of the foot switch with respect to the dental/medical professional will often change so that in most cases the dental/medical professional will also have to manually adjust the relative angular position of the foot switch in order to be able to access the whole functionality provided by the foot switch. Having to adjust the relative angular position of the foot switch each time the dental/medical professional changes his position with respect to the foot switch is cumbersome and distracts the dental/medical professional's attention from the patient.

US 2010/001948 A1 discloses a foot operated data entry/input pad with a plurality of foot-operated buttons. The foot buttons may be used to enter data values, such as numbers or symbols separately or in combination. Each button is preferably capable of entering different data values, preferably depending on the length of time that it is pressed or on the number of times that it is pressed in succession. A small controller may be included to allow the user to control the computer's pointer, allowing the user to switch between data entry fields.

The object of the present invention is to provide an improved control device and in particular an improved foot switch device that does not have the above outlined drawback. Moreover, the object of the present invention is to provide for a method of operating such an improved control device.

### SUMMARY OF THE INVENTION

The above object is achieved according to a first general aspect of the present invention by a control device according to claim 1 for actuating at least two functions of an instrument In communication with the control device. The control device comprises an actuation element that is configured to be actuated in at least two different ways. The control device itself is configured to be operated in a calibration mode and an actuation mode such that in the calibration mode a respective function of the at least two functions can be assigned to the at least two different ways of actuating the actuation element and such that in the actuation mode an actuation of the actuation element of the calibrated control element in either one of the at least two different ways of actuating the actuation element actuates or triggers the function assigned to the respective way of actuating the actuation element. In the calibration mode the control device determine its relative (angular) position relative to the user.

According to a preferred embodiment, in the calibration mode the assignment of a respective function of the at least two functions to the at least two different ways of actuating the actuation element is effected by the actuation element being actuated. Alternatively, the assignment of a respective function of the at least two functions to the at least two different ways of actuating the actuation element is effected by positioning a user's foot relative to the control device.

Preferably, the control device further comprises a base element floatingly supporting the actuation element such that a cavity is defined between the base element and the actuation element. Preferably, a plurality of actuation sensors is arranged within the cavity and the plurality of actuation sensors are configured to detect an actuation of the actuation element resulting in a motion of the actuation element towards the base element. According to a preferred embodiment, the actuation element is floatingly supported by the base element by at least one support element, such as a spring element, disposed on the base element. Advantageously, the base element substantially has the shape of a flat circle and/or the actuation element substantially has the shape of a radially symmetric plate turned upside down.

The control device can be connected via a cable and/or wirelessly to the instrument in communication with the control device.

According to a preferred embodiment the actuation element comprises or defines an actuation surface configured such that the at least two different ways of actuating the actuation element comprise the exertion of a force onto the actuation element at two different locations of the actuation surface.

The control device can be configured to operate in the calibration mode and the actuation mode simultaneously or alternately.

Preferably, the control device further comprises means for detecting any motion of the control device with respect to a supporting surface, preferably comprising a mouse ball configured to be in rolling contact with the floor surface.

According to a preferred embodiment the control device further comprises means for visually indicating to a user whether the control device Is operating in the calibration mode or the actuation mode and/or means for visually indicating to a user the different functions assigned to the actuation element. Preferably, the visual indication means comprise a plurality of LEDs corresponding in number and position to the plurality of actuation sensors.

According to a second aspect of the present invention a method of operating a control device for controlling at least two different functions of an instrument in communication with the control device is provided. The method comprises the steps of: calibrating the control device in a calibration mode by determining its relative position with respect to a user, and assigning the at least two different functions to at least two different ways of actuating an actuation element; and controlling the instrument in communication with the control device in an actuation mode to perform at least one of the at least two different functions defined in the calibration mode by an actuation of the actuation element. Preferably, the at least two different functions are assigned to the at least two different ways of actuating the actuation element by an actuation of the actuation element.

Additional advantages and features of the present invention are defined in the additional dependent claims and/or will become apparent by reference to the following detailed description and accompanying drawings.

### BRIEF DESCRIPTION OF THE DRAWINGS

FIGURE 1 shows a top sectional view of a preferred embodiment of a control device according to the present invention.
FIGURE 2 shows a cross-sectional view of the preferred embodiment of a control device according to the present invention along the line A-A of figure 1.

### DETAILED DESCRIPTION OF PREFERRED EMBODIMENTS

The present invention will now be further described by defining different aspects of the invention generally outlined above in more detail. Each aspect so defined may be combined with any other aspect or aspects unless clearly indicated to the contrary. In particular, any feature indicated as being preferred or advantageous may be combined with any other feature or features indicated as being preferred or advantageous.

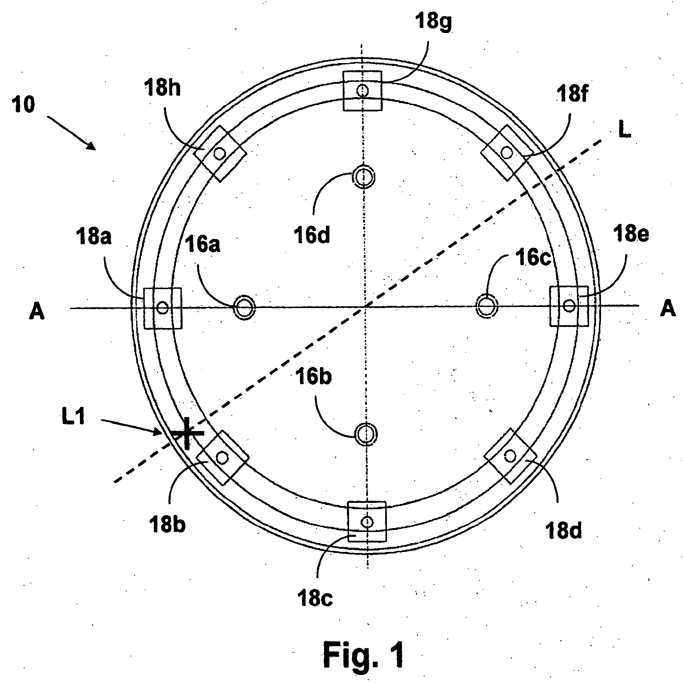

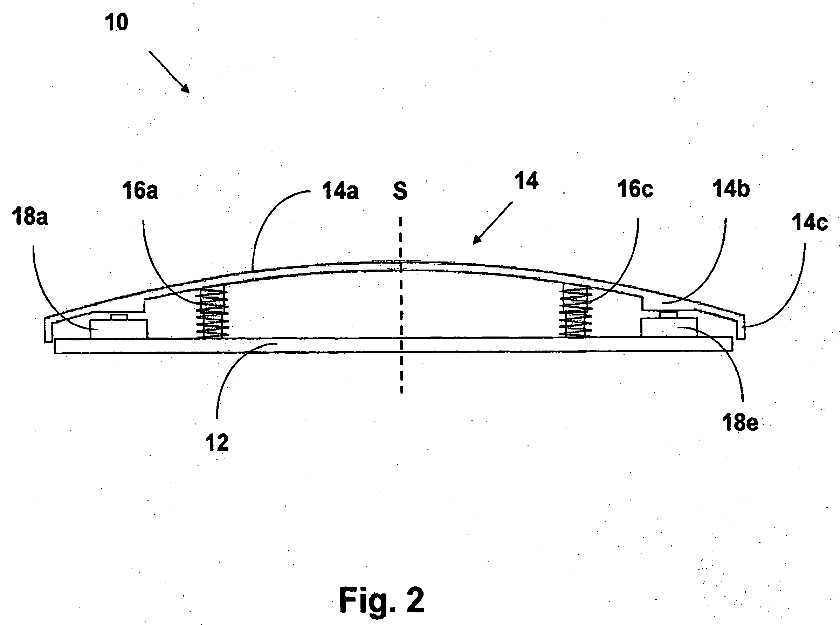

A preferred embodiment of a control device 10 according to present invention is shown in figures 1 and 2. The control device 10, which in particular can be operated by the foot of a user for controlling medical or dental equipment or instruments, comprises a base element 12 and an actuation element 14. As can be taken from the plan sectional view shown in figure 1, the control device 10 according to this preferred embodiment has a substantially circular, radially symmetric shape relative to a central symmetry axis S shown in figure 2. The base element 12 has substantially the shape of a flat circle and the actuation element 14 has substantially the shape of a plate turned upside down. As can be taken from figure 2, the top surface of the actuation element 14 defines an actuation surface 14a. Optionally, the bottom surface of the base element 12 can be outfitted with a non-skid, rubber backing to facilitate keeping the base element 12 and, thus, the control device 10 in place on the floor.

The base element 12 and the actuation element 14 are preferably connected by means of four support elements 16a-d, e.g. spring elements, such that the actuation element 14 is floatingly supported by the base element 12. In this floating configuration the actuation element 14 can be depressed towards the base element 12, for instance, by the foot of a user exerting a force on the actuation surface 14a of the actuation element 14. Such a depression will lead to a deformation of some or all of the support elements 16a-d, depending on the exact position of the force exerted by the user on the actuation surface 14a of the actuation element 14. Once this force is no longer being exerted, the actuation element 14 will move back into its default position due to the restoring force exerted by the biased springs of the support elements 16a-d. As can be taken from the plan view shown In figure 1, in the preferred embodiment four support elements 16a-d are symmetrically distributed around the central symmetry axis S of the control device 10. However, the person skilled in the art will appreciate that different arrangements of support elements as well as more or less than four support elements are also possible according to the present invention. Moreover, the person skilled in the art will appreciate that other means than spring elements can be used as support elements to support the actuation element 14 as well.

As can be taken from figure 2, due to the floating support of the actuation element 14 by the base element 12 and the support elements 16a-d a cavity is defined between the base element 12 and the actuation element 14. A plurality of actuation sensors 18a-h are arranged within the cavity defined between the base element 12 and the actuation element 14 for sensing any actuation of the actuation element 14, i.e. for sensing any substantial movement of the actuation element 14 towards the base element 12. Preferably, the actuation sensors 18ah are disposed on the base element 12 near the peripheral edge thereof. As can be taken from the plan view shown in figure 1, in the preferred embodiment eight actuation sensors 18a-h are symmetrically distributed around the central symmetry axis S of the control device 10. Preferably, each actuation sensor 18ah comprises a retractable pin that is biased, for instance, by means of a respective spring element Into an extended position. In this extended position the tip of the pin of each actuation sensor 18a-h is preferably In close contact or almost abutting relationship with the bottom surface of the actuation element 14. For instance, in the embodiment shown in figure 2, the tip of each pin of each sensor 18a-h almost abuts an annular ledge or shoulder portion 14b being defined by the bottom surface of the actuation element 14. Moreover, the actuation element 14 can comprise a circumferential, annular skirt portion 14c for preventing the accumulation of any dust or dirt within the cavity defined between the base element 12 and the actuation element 14.

As the person skilled in the art will appreciate, an actuation of the actuation element 14 by a user exerting a force on the actuation surface 14a of the actuation element 14 will have the effect that depending on the exact location of the exertion of the force at least one, some or all of the pins of the actuation sensors 18a-h because of their abutting relationship with the bottom surface of the actuation element 14 will be displaced from their extended position(s) towards their retracted position(s). Thus, this motion will be sensed or registered by at least one, some or all of the actuation sensors 18a-h. An activation of the actuation sensors 18a-h in this manner will trigger a corresponding actuation signal that can be communicated to an instrument In communication with the control device 10 and/or to an Internal control unit (not shown) of the control device 10 for processing the actuation signals provided by the actuations sensors 18a-h and communicating resulting control signals to the instrument in communication with the control device 10.

The person skilled in the art will appreciate that the number, configuration and arrangement of the above described actuation sensors 18a-h corresponds to a preferred embodiment and a different number of differently arranged actuation sensors having a different configuration could be used as well. For instance, according to the present invention it would be possible that the actuation sensors are configured to sense the magnetic fields produced by magnetic elements disposed in or on the actuation element so that an actuation of the actuation element will result in a different magnetic field sensed by the actuations sensors and the triggering of a corresponding actuation signal.

According to the present Invention the control device 10 is configured to operate in two modes, namely a calibration or gauge mode on the one hand and an actuation or control mode on the other hand. Essentially, In the calibration or gauge mode the control device 10 is configured to determine its relative (angular) position with respect to the user and calibrate itself accordingly. The person skilled in the art will appreciate from the following detailed description that the details of these two different modes of operation can be implemented in the control device 10 in a number of different ways. For instance, according to certain embodiments of the present invention the control device 10 can be configured to change from the calibration or gauge mode to the actuation or control mode by an appropriate actuation of the actuation element 14. Alternatively, according to further embodiments of the present invention the control device 10 can operate in both modes simultaneously, wherein a calibration of the control device 10 is achieved by means of a first way of actuating the actuation element 14, such as by depressing the actuation element 14 for more than 5 seconds, and an actuation of the control device 10 is achieved by means of a second different way of actuating the actuation element 14, such as by depressing the actuation element 14 for less than 5 seconds. One preferred embodiment will now be described in the context of figures 1 and 2.

Preferably, in the actuation or control mode the control device 10 acts as a multifunctional switch, i.e. a switch that can control or trigger multiple functions of an instrument In communication with the control device 10, wherein the different functions of the instrument in communication with the control device 10 are assigned to individual actuation sensors 18a-h or groups of adjacent actuation sensors 18a-h so that an actuation of the actuation element 14 via its actuation surface 14a at or close to the position of a certain actuation sensor 18a-h will trigger the function assigned to this actuation sensor or the group of actuation sensors this actuation sensor belongs to. For example, In the preferred embodiment shown in figures 1 and 2 four of the eight actuation sensors 18a-h, such as the actuation sensors 18a-d, could be assigned to a first function, whereas the other four of the eight actuation sensors, such as the actuation sensors 18e-h, could be assigned to a second function of an instrument in communication with and to be controlled by the control device 10. In other words, an actuation of at least one, some or all of the pins of the actuation sensors 18a-d by a user exerting a force on the actuation surface 14a of the actuation element 14 somewhere in the semicircular region defined by the actuation sensors 18a-d will trigger the first function assigned to these actuation sensors 18a-d. Likewise, an actuation of at least one, some or all of the pins of the actuation sensors 18e-h by a user exerting a force on the actuation surface 14a of the actuation element 14 somewhere in the semicircular region defined by the actuation sensors 18e-h will trigger the second function assigned to these actuation sensors 18e-h. As described above, the first and the second function could relate to two different functions of one instrument controlled by the control device 10. Alternatively, the first and the second function could relate to the operation of a first instrument and a second Instrument in communication with and controlled by the control device 10.

In the calibration or gauge mode the control device 10 is configured to determine its relative (angular) position with respect to the user and assign specific control functions to respective ones of the actuation sensors 18a-h. Such an assignment of the actuation sensors 18a-h to specific functions to be controlled by the control device 10 requires the interaction with the user. Preferably, in the calibration or gauge mode of the control device 10 a depression of the actuation element 14 towards the base element 12 by the user's foot exerting a force onto the actuation surface 14a of the actuation element 14 near the circumferential edge thereof will lead to such an assignment of the actuation sensors 18a-h to specific functions, i.e. to a calibration of the control device 10, according to the preferred calibration mechanism described below. Alternatively, the control device 10 could comprise additional sensors for detecting the position (and/or motion) of the user's foot such that only by means of the position of the user's foot, i.e. the location of the user's foot above the actuation surface 14a of the actuation element 14, the control device 10 can be calibrated (i.e. its relative position to the user's foot can be determined and the specific control functions can be assigned to the actuation sensors 18a-h accordingly).

According to a preferred embodiment the control device 10 shown in figures 1 and 2 acts as a dual function switch, i.e. a switch that can control at least two different functions of an instrument in communication with the control device 10. In case the user exerts a force onto the actuation surface 14a of the actuation element 14 near a circumferential edge thereof at a position that in the plan view of figure 1 lies between two actuation sensors, such as at the exemplary position L1 indicated In figure 1 by a cross, which lies between the actuation sensors 18a and 18b, the actuation sensors 18a-h will be assigned to different instrument functions or calibrated as follows. The actuation sensors lying on one side of the notional line L running in the plan view of figure 1 from the point L1 through the center of the control device 10 will be assigned to a first function of the instrument in communication with the control device 10, whereas the actuation sensors lying on the other side of this notional line L will be assigned to a second function thereof. For example, in the embodiment shown in figure 1 the actuation sensors 18a and 18f-h will be assigned to the first function of the instrument in communication with the control device 10, whereas the actuation sensors 18b-e will be assigned to the second function thereof. In the rather unlikely case that a user will exert a force directly above an actuation sensor so that the notional line L in the exemplary embodiment of figure 1 runs through two actuation sensors, one of these actuation sensors can be assigned to the first function of the instrument in communication with the control device 10 and the other one can be assigned to the second function thereof.

The person skilled in the art will appreciate that the radial symmetric arrangement of eight actuation sensors 18a-h corresponds to a preferred exemplary embodiment. The present invention can also be implemented with more or less actuation sensors as well as with different arrangements of actuation sensors. It is contemplated, for instance, that arrangements of actuation sensors can be implemented according to the present invention where any notional line running in a plan view through one actuation sensor and the center of the control device does not run through another actuation sensor, as is the case in the embodiment shown in figure 1. Moreover, the person skilled in the art will appreciate that according to the present invention calibration mechanisms similar to the above can be used to assign more than two functions of an instrument in communication with the control device 10 to the actuation sensors 18a-h thereof. For instance, it might be possible to use a first notional line running through the point of exertion of force and the center of the control device 10 as well as a second notional line that is perpendicular thereto and also runs through the center of the control device to define four quarter sections of the control device 10 corresponding to four different functions of an instrument to be controlled thereby.

Once the control device 10 has been calibrated, for instance by means of the above described preferred calibration mechanism, according to certain preferred embodiments the control device 10 will no longer operate in the calibration or gauge mode but In the control or actuation mode. For instance, In a control device 10 that has been calibrated as indicted in figure 1, i.e. by exerting a force onto the actuation surface 14a of the actuation element 14 at the position L1 and thereby assigning the actuation sensors 18a and 18f-h to a first function of an instrument in communication with the control device 10 and the actuation sensors 18b-e to a second function thereof, an exertion of a force on the actuation surface 14a of the actuation element 14 on the from the user's point of view "left side" of the notional line L in the plan view of figure 1 will lead to a triggering of the first function of the instrument in communication with the control device 10, whereas an exertion of a force on the actuation surface 14a of the actuation element 14 on the "right side" of the notional line L In the plan view of figure 1 will lead to a triggering of the second function of the instrument In communication with the control device 10.

As already described above, according to alternative preferred embodiments of the present invention the control device 10 can operate in both modes, i.e. on the one hand the calibration or gauge mode and on the other hand the control or actuation mode, simultaneously, wherein a calibration of the control device 10 is achieved by means of a first way of actuating the actuation element 14, such as by depressing the actuation element 14 at any position of the actuation surface 14a for more than 5 seconds, and an actuation of the control device 10 is achieved by means of a second different way of actuating the actuation surface of the actuation element 14, such as by depressing the actuation element 14 for less than 5 seconds. The person skilled in the art will appreciate that also in these alternative embodiments a calibration and actuation mechanism as described above can be implemented in the control device 10. For Instance, exerting a force for more than 5 seconds at the position of the actuation surface 14a of the actuation element 14 marked L1 in figure 1 preferably results in an assignment of the actuation sensors lying on the "left side" of the notional line L running in the plan view of figure 1 from the point L1 through the center of the control device 10, i.e. the actuation sensors 18a and 18f-h, to a first function of an instrument in communication with the control device 10 and an assignment of the actuation sensors lying on the "right side" of this notional line L, i.e. the actuation sensors 18b-e, to a second function thereof. Having calibrated the control device 10 in such a way, an exertion of a force for less than 5 seconds on the actuation surface 14a of the actuation element 14 on the "left side" of the notional line L in the plan view of figure 1 will lead to a triggering of the first function of the instrument in communication with the control device 10, whereas an exertion of a force for less than 5 seconds on the actuation surface 14a of the actuation element 14 on the "right side" of the notional line L in the plan view of figure 1 will lead to a triggering of the second function of the instrument in communication with the control device 10. An exertion of a force for more than 5 seconds on the actuation surface 14a of the actuation element 14 will result in another calibration of the control device, for instance, according to the above described preferred calibration mechanism.

In the preferred embodiments, where the control device 10 operates alternatively in the calibration or gauge mode on the one hand and in the control or actuation mode on the other hand the control device 10 is preferably configured to remain in the control or actuation mode as long as the control device 10 does not change its position and, thus, its angular position relative to the user. To this end, the control device 10 in these preferred embodiments furthermore preferably comprises means for detecting any change of position of the control device 10. Any substantial change of position of the control device 10 detected by these means will result in a transition to the calibration or gauge mode. Preferred means for detecting any change of position of the control device 10 comprise at least one ball rotatably supported within the base element 12 substantially functioning like a computer mouse ball. However, the person skilled In the art is well aware of various other means that could be used for detecting any change of position of the control device 10, such as infrared laser diodes as used in an optical computer mouse or any other motion sensor.

In the preferred embodiments, where the control device 10 operates alternatively in the calibration or gauge mode on the one hand and in the control or actuation mode on the other hand, the control device 10 furthermore can comprise means for visually indicating to a user whether the control device 10 currently operates in the calibration or gauge mode or in the control or actuation mode. Preferably, a plurality of LEDs are provided on the actuation surface 14a of the actuation element 14 at positions that correspond to and are aligned with the positions of the actuation sensors 18a-h on the base element 12 such that each LED corresponds to an actuation sensor. These LEDs not only allow the user to determine the position of a sensor 18a-h located within the cavity defined between the base element 12 and the actuation element 14, but also indicate whether the control device 10 currently operates in the calibration or gauge mode or in the control or actuation mode. For instance, in the calibration or gauge mode the control device 10 could be configured such that all LEDs blink concurrently at certain intervals indicating to the user that the control device 10 has not been calibrated yet and is ready for calibration. After a calibration of the control device 10 according to the preferred calibration mechanism described further above, wherein by exerting at the exemplary position of the actuation surface 14a of the actuation element 14 marked L1 in figure 1 the actuation sensors lying on the "left side" of the notional line L running in the plan view of figure 1 from the point L1 through the center of the control device 10, i.e. the actuation sensors 18a and 18f-h, have been assigned to a first function of an Instrument in communication with the control device 10 and the actuation sensors lying on the "right side" of this notional line L, i.e. the actuation sensors 18b-e, have been assigned to a second function thereof, the control device 10 could be configured such that the LEDs corresponding to the actuation sensors lying on the "left side" of the notional line L, i.e. the actuation sensors 18a and 18f-h, emit light according to a different temporal pattern than the LEDs corresponding to the actuation sensors lying on the "right side" of this notional line L, i.e. the actuation sensors 18b-e. For instance, the LEDs corresponding to the actuation sensors 18a and 18f-h and the LEDs corresponding to the actuation sensors 18b-e could emit light pulses alternately or one of these groups of LEDs could emit light constantly, whereas the other group of LEDs does not emit any light. Such configurations of the control device 10 in the control or actuation mode help the user to discern which parts of the actuation surface 14a of the actuation element 14 he or she has to actuate in order to trigger the various functions of an instrument in communication with and controlled by the control device 10.

The control device 10 of this invention may be used to control the operation of various instruments and machines, such as electrocardiogram machines, X-ray machines, surgical cutting instruments, endoscopic and laproscopic tools, blood analyzers, diagnostic tools, dental chairs, dental irrigators, dental air polishing and prophylaxis systems, dental drills, endodontic and periodontic handpleces, and other dental equipment. Preferably, the control device 10 of this invention is configured as a foot control device.

Preferably, the control device 10 is used to operate a dental/medical instrument in a wireless, remote control system. In such a system, the control device 10 may include a transmitter or transceiver that transmits a radio frequency (RF) signal to a RF receiver In an optional base unit of the dental/medical instrument, which receives the signal. Wireless information including, for example, identification codes, equipment status, alarm messages, and the like may be sent back and forth between the control device 10 and the dental/medical instrument using such an RF transceiver. It is recognized that wireless communication systems, other than RF systems, could be used. For example, infrared or ultrasound communication systems could be used.

Alternatively, the control device 10 according to the present invention may be used to operate a dental/medical instrument in a hard-wired system. In such a system, the control device 10 is connected to an optional base unit of the dental/medical instrument by a connector cable extending from the control device 10. The control signals are sent from the control device 10 or its optional control unit to the dental/medical instrument or its optional base unit via the connector cable.

The present invention as described in detail above is not limited to the particular devices, uses and methodology described as these may vary. For instance, although the present invention has been described above in the context of a preferred embodiment of a foot switch, it can also be applied advantageously to switches operated by other means, such as the hands of a user. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

## Claims

1. A control device (10) for controlling at least two different functions of an instrument in communication with the control device (10), the control device (10) comprising an actuation element (14) configured to be actuated in at least two different ways,
wherein the control device (10) is configured to be operated in a calibration mode and an actuation mode,
such that in the calibration mode the control device (10) is configured to determine its relative position with respect to a user, wherein in the calibration mode a respective function of the at least two functions can be assigned to the at least two different ways of actuating the actuation element (14), and
such that in the actuation mode an actuation of the actuation element (14) of the calibrated control element (10) in either one of the at least two different ways of actuating the actuation element (14) actuates the function assigned to the respective way of actuating the actuation element (14).

2. The control device (10) of claim 1, wherein in the calibration mode the assignment of a respective function of the at least two functions to the at least two different ways of actuating the actuation element (14) is effected by an actuation of the actuation element (14).

3. The control device (10) of claim 1, wherein in the calibration mode the assignment of a respective function of the at least two functions to the at least two different ways of actuating the actuation element (14) is effected by positioning a user's foot relative to the control device (10).

4. The control device (10) of claim 1, wherein the control device further comprises a base element (12) floatingly supporting the actuation element (14).

5. The control device (10) of claim 4, wherein a cavity is defined between the base element (12) and the actuation element (14) and a plurality of actuation sensors (18ah) is arranged within the cavity, wherein the plurality of actuation sensors (18a-h) are configured to detect an actuation of the actuation element (14) resulting in a motion of the actuation element (14) towards the base element (12).

6. The control device (10) of claims 4 or 5, wherein the actuation element (14) is floatingly supported by at least one support element (16a-d), such as a spring element.

7. The control device (10) of claims 4 or 5, wherein the base element (12) substantially has the shape of a flat circle and/or the actuation element (14) substantially has the shape of a radially symmetric plate turned upside down.

8. The control device (10) of claim 1, wherein the control device (10) is connected via a cable and/or wirelessly to the instrument in communication with the control device (10).

9. The control device (10) of claim 1, wherein the actuation element (14) defines an actuation surface (14a) configured such that the at least two different ways of actuating the actuation element (14) comprise the exertion of a force onto the actuation element (14) at two different locations of the actuation surface (14a).

10. The control device (10) of claim 1, wherein the control device (10) is configured to operate in the calibration mode and the actuation mode simultaneously or alternately.

11. The control device (10) of claim 1, further comprising means for detecting any motion of the control device (10) with respect to a supporting surface, preferably comprising a mouse ball.

12. The control device (10) of claim 1, wherein the control device (10) further comprises means for visually indicating to a user whether the control device is operating in the calibration mode or the actuation mode and/or means for visually indicating to a user the different functions assigned to the actuation element (14).

13. The control device (10) of claim 12, wherein the visual indication means comprise a plurality of LEDs corresponding in number and position to the plurality of actuation sensors (18a-h).

14. A method of operating a control device (10) for controlling at least two different functions of an instrument in communication with the control device (10), the control device comprising an actuation element (14) configured to be actuated in at least two different ways, the method comprising the following steps:
calibrating the control device (10) in a calibration mode by
- determining its relative position with respect to a user, and
- assigning the at least two different functions to the at least two different ways of actuating the actuation element (14); and
controlling the instrument in communication with the control device (10) in an actuation mode to perform at least one of the at least two different functions defined in the calibration mode by an actuation of the actuation element (14).

15. The method of claim 14, wherein the at least two different functions are assigned to the at least two different ways of actuating the actuation element (14) by an actuation of the actuation element (14).

## Patentansprüche

1. Steuerungsvorrichtung (10) zum Steuern von mindestens zwei verschiedenen Funktionen eines Instruments, das in Kommunikation mit der Steuerungsvorrichtung (10) ist, wobei die Steuerungsvorrichtung (10) ein Betätigungselement (14) umfasst, das ausgebildet ist, auf mindestens zwei verschiedene Weisen betätigt zu werden,
wobei die Steuerungsvorrichtung (10) ausgebildet ist, in einem Kalibricrungsmodus und einem Betätigungsmodus betrieben zu werden,
so dass die Steuerungsvorrichtung (10) im Kalibrierungsmodus ausgebildet ist, ihre relative Position in Bezug auf einen Anwender festzustellen, wobei im Kalibrierungsmodus eine entsprechende Funktion der mindestens zwei Funktionen den mindestens zwei verschiedenen Weisen des Betätigens des Betätigungselements (14) zugeordnet werden kann, und
so dass eine Betätigung des Betätigungselements (14) der kalibrierten Steuerungsvorrichtung (10) im Betätigungsmodus in einer jeglichen der mindestens zwei verschiedenen Weisen des Betätigens des Betätigungselements (14) die Funktion auslöst, die der entsprechenden Weise des Betätigens des Betätigungselements (14) zugeordnet ist.

2. Steuerungsvorrichtung (10) nach Anspruch 1, wobei die Zuordnung einer entsprechenden Funktion der mindestens zwei Funktionen zu den mindestens zwei verschiedenen Weisen des Betätigens des Betätigungselements (14) im Kalibrierungsmodus durch eine Betätigung des Betätigungselements (14) bewirkt wird.

3. Steuerungsvorrichtung (10) nach Anspruch 1, wobei die Zuordnung einer entsprechenden Funktion der mindestens zwei Funktionen zu den mindestens zwei verschiedenen Weisen des Betätigens des Betätigungselements (14) im Kalibrierungsmodus durch Positionieren eines Fußes eines Anwenders relativ zu der Steuerungsvorrichtung (10) bewirkt wird.

4. Steuerungsvorrichtung (10) nach Anspruch 1, wobei die Steuerungsvorrichtung ferner ein Basiselement (12) umfasst, das das Betätigungselement (14) schwimmend unterstützt.

5. Steuerungsvorrichtung (10) nach Anspruch 4, wobei eine Kavität zwischen dem Basiselement (12) und dem Betätigungselement (14) definiert ist und eine Vielzahl von Betätigungssensoren (18a-h) in der Kavität angeordnet sind, wobei die Vielzahl der Betätigungssensoren (18a-h) ausgebildet sind, eine Betätigung des Betätigungselements (14) zu detektieren, was in einer Bewegung des Betätigungselements (14) in Richtung des Basiselements (12) resultiert.

6. Steuerungsvorrichtung (10) nach Anspruch 4 oder 5, wobei das Betätigungselement (14) schwimmend durch mindestens ein Unterstützungselement (16a-d), wie z.B. ein Federelement, unterstützt ist.

7. Steuerungsvorrichtung (10) nach Anspruch 4 oder 5, wobei das Basiselement (12) im Wesentlichen die Form eines flachen Rings und/oder das Betätigungselement (14) im Wesentlichen die Form einer radial-symmetrischen Platte, die auf den Kopf gestellt ist, aufweist.

8. Steuerungsvorrichtung (10) nach Anspruch 1, wobei die Steuerungsvorrichtung (10) über ein Kabel und/oder drahtlos mit dem Instrument, das in Kommunikation mit der Steuerungsvorrichtung (10) ist, verbunden ist.

9. Steuerungsvorrichtung (10) nach Anspruch 1, wobei das Betätigungselement (14) eine Betätigungsfläche (14a) definiert, die so ausgebildet ist, dass die mindestens zwei verschiedenen Weisen des Betätigens des Betätigungselements (14) die Ausübung einer Kraft auf das Betätigungselement (14) an zwei verschiedenen Stellen der Betätigungsfläche (14a) umfasst.

10. Steuerungsvorrichtung (10) nach Anspruch 1, wobei die Steuerungsvorrichtung (10) ausgebildet ist, in dem Kalibrierungsmodus und dem Betätigungsmodus simultan oder alternierend zu arbeiten.

11. Steuerungsvorrichtung (10) nach Anspruch 1, ferner umfassend Mittel zum Detektieren einer jeglichen Bewegung der Steuerungsvorrichtung (10) in Bezug auf eine Stützfläche, vorzugsweise umfassend eine Mauskugel.

12. Steuerungsvorrichtung (10) nach Anspruch 1, wobei die Steuerungsvorrichtung (10) ferner Mittel, die einem Anwender visuell anzeigen, ob die Steuerungsvorrichtung im Kalibrierungsmodus oder im Betätigungsmodus arbeitet und/oder Mittel, die einem Anwender die verschiedenen Funktionen, die dem Betätigungselement (14) zugeordnet sind, visuell anzeigen, umfasst.

13. Steuerungsvorrichtung (10) nach Anspruch 12, wobei die Mittel für die visuelle Anzeige eine Vielzahl von LEDs umfassen, die in Anzahl und Position der Vielzahl von Betätigungssensoren (18a-h) entsprechen.

14. Verfahren zum Betreiben einer Steuerungsvorrichtung (10) zum Steuern von mindestens zwei verschiedenen Funktionen eines Instruments, das in Kommunikation mit der Steuerungsvorrichtung (10) ist, wobei die Steuerungsvorrichtung ein Betätigungselement (14) umfasst, das ausgebildet ist, auf mindestens zwei verschiedene Weisen betätigt zu werden, wobei das Verfahren die folgenden Schritte umfasst:
Kalibrieren der Steuerungsvorrichtung (10) in einem Kalibrierungsmodus durch
- Feststellen ihrer relativen Position in Bezug auf einen Anwender, und
- Zuordnen der mindesten zwei verschiedenen Funktionen zu den mindestens zwei verschiedenen Weisen des Betätigens des Betätigungselements (14); und
Steuern des Instruments, das in Kommunikation mit der Steuerungsvorrichtung (10) ist, in einem Betätigungsmodus, um mindesten eine der mindestens zwei verschiedenen Funktionen, die in dem Kalibrierungsmodus definiert werden, durch eine Betätigung des Betätigungselements (14) auszuführen.

15. Verfahren nach Anspruch 14, wobei die mindestens zwei verschiedenen Funktionen den mindestens zwei verschiedenen Weisen des Betätigens des Betätigungselements (14) durch eine Betätigung des Betätigungselements (14) zugeordnet werden.

## Revendications

1. Dispositif de commande (10) pour commander au moins deux fonctions différentes d'un instrument en communication avec le dispositif de commande (10), le dispositif de commande (10) comprenant un élément d'actionnement (14) configuré pour être actionné d'au moins deux manières différentes, tandis que le dispositif de commande (10) est configuré pour être mis en oeuvre dans un mode étalonnage et un mode actionnement,
de telle façon que dans le mode étalonnage le dispositif de commande (10) est configuré pour déterminer sa position relative par rapport à un utilisateur, tandis que dans le mode étalonnage une fonction respective des au moins deux fonctions peut être assignée aux au moins deux façons différentes d'actionnement de l'élément d'actionnement (14), et
de telle façon que dans le mode actionnement un actionnement de l'élément d'actionnement (14) de l'élément de commande (10) étalonné actionne, selon l'une ou l'autre des au moins deux façons d'actionnement différentes l'élément d'actionnement (14), la fonction assignée à la façon respective d'actionnement de l'élément d'actionnement (14).

2. Dispositif de commande (10) selon la revendication 1, dans lequel dans le mode étalonnage l'affectation d'une fonction respective parmi les au moins deux fonctions aux au moins deux façons différentes d'actionnement de l'élément d'actionnement (14) est effectuée par actionnement de l'élément d'actionnement (14).

3. Dispositif de commande (10) selon la revendication 1, dans lequel dans le mode étalonnage l'affectation d'une fonction respective parmi les au moins deux fonctions aux au moins deux façons différentes d'actionnement de l'élément d'actionnement (14) est effectuée par positionnement d'un pied de l'utilisateur par rapport au dispositif de commande (10).

4. Dispositif de commande (10) selon la revendication 1, dans lequel le dispositif de commande comprend en outre un élément de base (12) supportant de manière flottante l'élément d'actionnement (14).

5. Dispositif de commande (10) selon la revendication 4, dans lequel une cavité est définie entre l'élément de base (12) et l'élément d'actionnement (14) et une pluralité de détecteurs d'actionnement (18a-h) est disposée dans la cavité, la pluralité de détecteurs d'actionnement (18a-h) étant configurée pour détecter un actionnement de l'élément d'actionnement (14) ayant pour résultat un déplacement de l'élément d'actionnement (14) en direction de l'élément de base (12).

6. Dispositif de commande (10) selon les revendications 4 ou 5, dans lequel l'élément d'actionnement (14) est supporté en flottement par au moins un élément support (16a-d), tel qu'un élément ressort.

7. Dispositif de commande (10) selon les revendications 4 ou 5, dans lequel l'élément de base (12) a en substance la forme d'un cercle plat et/ou l'élément d'actionnement (14) a en substance la forme d'une assiette à symétrie radiale, retournée.

8. Dispositif de commande (10) selon la revendication 1, dans lequel le dispositif de commande (10) est connecté via un câble et/ou par une liaison sans fil à l'instrument en communication avec le dispositif de commande (10).

9. Dispositif de commande (10) selon la revendication 1, dans lequel le dispositif d'actionnement (14) définit une surface d'actionnement (14a) configurée de telle façon que les au moins deux façons différentes d'actionnement de l'élément d'actionnement (14) comprennent l'exercice d'une force sur l'élément d'actionnement (14) en deux endroits différents de la surface d'actionnement (14a).

10. Dispositif de commande (10) selon la revendication 1, dans lequel le dispositif de commande (10) est configuré pour opérer en mode étalonnage et en mode actionnement simultanément ou alternativement.

11. Dispositif de commande (10) selon la revendication 1, comprenant en outre des moyens pour la détection de tout mouvement du dispositif de commande (10) par rapport à la surface de support, de préférence comprenant une boule de souris.

12. Dispositif de commande (10) selon la revendication 1, dans lequel le dispositif de commande (10) comprend en outre des moyens pour indiquer visuellement à un utilisateur si le dispositif de commande est en fonctionnement en mode étalonnage ou en mode actionnement et/ou des moyens pour indiquer visuellement à un utilisateur les différentes fonctions assignées à l'élément d'actionnement (14).

13. Dispositif de commande (10) selon la revendication 12, dans lequel les moyens d'indication visuelle comprennent une pluralité de LEDs correspondant en nombre et position à la pluralité de détecteurs d'actionnement (18a-h).

14. Procédé pour la mise en fonctionnement d'un dispositif de commande (10) pour la commande d'au moins deux fonctions différentes d'un instrument en communication avec le dispositif de commande (10), le dispositif de commande comprenant un élément d'actionnement (14) configuré pour être actionné d'au moins deux façons différentes, le procédé comportant les étapes suivantes:
étalonnage du dispositif de commande (10) dans un mode étalonnage par
- détermination de sa position relative par rapport à un utilisateur, et
- attribution des au moins deux fonctions différentes aux au moins deux façons différentes d'actionnement de l'élément d'actionnement (14); et
commande de l'instrument en communication avec le dispositif de commande (10) dans un mode d'actionnement destiné à réaliser au moins l'une des au moins deux fonctions différentes définies dans le mode étalonnage par actionnement de l'élément d'actionnement (14).

15. Procédé selon la revendication 14, dans lequel les au moins deux fonctions différentes sont attribuées aux au moins deux façons différentes d'actionnement de l'élément d'actionnement (14) par une mise en fonctionnement de l'élément d'actionnement (14).
